# EUROPÄISCHE PATENTSCHRIFT

(11) **EP 2 452 616 B1**
(45) Veröffentlichungstag und Bekanntmachung des Hinweises auf die Patenterteilung: **04.09.2019**
(21) Anmeldenummer: 11183927.0
(22) Anmeldetag: 18.07.2005
(51) Int. Cl.: A61B 3/13, A61B 90/30

(54) **Beobachtungseinrichtung**
Observation device
Dispositif d'observation

(30) Priorität: 06.08.2004 DE 102004038372; 18.10.2004 DE 102004050651
(43) Veröffentlichungstag der Anmeldung: 16.05.2012
(62) Teilanmeldung aus: 05766830.3
(73) Patentinhaber: Carl Zeiss Meditec AG, 07745 Jena (DE)
(72) Erfinder: Reimer, Peter, 73479 Ellwangen (DE); Abramowsky, Heinz, 89537 Geigen (Brenz) (DE); Kolster, Daniel, 73447 Oberkochen (DE); Strähle Dr., Fritz, 73540 Heubach (DE); Abele, Alfons, 73527 Schwäbisch-Gmünd (DE)
(74) Vertreter: Müller & Schubert

(56) Entgegenhaltungen:
- DE-A1- 4 326 761
- DE-A1- 4 417 273

## Beschreibung

Die vorliegende Erfindung betrifft ein Operationsmikroskop für die Ophthalmo-Chirurgie.

Ein ophthalmologisches Operationsmikroskop wird beispielsweise für eine spezielle Anwendung in der Augenchirurgie eingesetzt, nämlich der Kataraktchirurgie.

Bei der Kataraktchirurgie wird eine - beispielsweise durch den grauen Star getrübte - Augenlinse durch eine Kunstlinse ersetzt.

Die Augenlinse eines Auges befindet sich in einer dünnen Umhüllung, der so genannten Linsenkapsel. Zur Entfernung der Augenlinse wird durch einen dünnen Schnitt in die Linsenkapsel ein Zugang zur Augenlinse geschaffen und die Augenlinse mit einem mikrochirurgischen Gerät zunächst in kleine Einzelstücke zerteilt, die dann mittels einer Absaugvorrichtung entfernt werden.

Dieser Vorgang findet unter mikroskopischer Beobachtung - beispielsweise unter stereomikroskopischer Beobachtung - unter Einsatz einer für solche Eingriffe speziell ausgelegten Beleuchtungseinrichtung statt. Diese Beleuchtungseinrichtung stellt sowohl eine für die Ausleuchtung des gesamten Operationsfelds notwendige Umfeldbeleuchtung als auch eine für die Kataraktoperation entscheidend wichtige rote Hintergrundbeleuchtung für das eigentliche auf den Pupillenbereich der Augenlinse begrenzte Operationsfeld dar. Diese rote Hintergrundbeleuchtung rührt von dem Anteil des Beleuchtungslichts her, der über die transparenten Augenmedien schließlich auf die wegen einer guten Durchblutung rot erscheinenden Netzhaut trifft, von dieser zurückgestreut wird und dann natürlich über das Operationsmikroskop auch vom Chirurgen als rot erscheinende Hintergrundbeleuchtung beobachtet werden kann. Diese in der Kataraktchirurgie ganz charakteristische rote Hintergrundbeleuchtung ist in Fachkreisen allgemein unter dem Begriff "roter Reflex" bekannt.

Für eine optimale Erkennung der für die Kataraktoperation relevanten Details erweist sich für den Operateur eine möglichst homogene rote Hintergrundbeleuchtung als eine notwendige Voraussetzung. Eine erste Anforderung an die Beleuchtungseinrichtung ist also eine möglichst gute Homogenität des roten Reflexes über die gesamte Patientenpupille zu gewährleisten.

Zur vollständigen Beseitigung der Linsenreste der in winzige Teilstücke zerkleinerten Augenlinse und zur guten Erkennung von durchsichtigen Membranen, beispielsweise von der Linsenkapsel, muss eine weitere Anforderung erfüllt werden, nämlich eine gute Kontrastierung von Phasenobjekten und zwar möglichst auch über die gesamte Patientenpupille.

In der Vergangenheit sind im Zusammenhang mit der Erzeugung einer solchen roten Hintergrundbeleuchtung bereits verschiedene Lösungen bekannt geworden.

In der US-A-4,779,968 ist eine koaxiale Beleuchtung für ein Operationsmikroskop beschrieben. Gemäß dieser Lösung ist ein Beleuchtungsmodul vorgesehen, das als Zusatzbaustein an vorhandene Operationsmikroskope nachträglich eingebaut werden kann. Dieser Zusatzbaustein wird vorzugsweise objektseitig unterhalb des Hauptobjektivs der Beobachtungseinrichtung angebracht. Die Beleuchtungseinkopplung auf der Mikroskopachse erfolgt entweder mit einer Teilerplatte oder einem Teilerwürfel.

In der DE 40 28 605 C2 ist eine Beleuchtungseinrichtung für ein Operationsmikroskop beschrieben, welches eine Kombination von Null-Grad-, Koaxial- und Schrägbeleuchtung zulässt. Dazu verfügt die Beleuchtungseinrichtung über verschiebbare Teilspiegel sowie einen festen Sechs-Grad-Spiegel samt den jeweiligen variierbaren Blenden, womit der Beleuchtungswinkel und die Lichtanteile der jeweiligen Beleuchtungsrichtung variiert werden können. Der Schwerpunkt dieser bekannten Lösung liegt in der Kontraststeigerung mittels einer Koaxialbeleuchtung, wobei es sich bei der Koaxialbeleuchtung um eine achsnahe Schrägbeleuchtung handelt.

In der DE 196 38 263 A1 ist ein ophthalmologisches Beobachtungsgerät offenbart, bei dem der bei Beleuchtung eines Patientenauges zur Beobachtung der vorderen Augenabschnitte unvermeidliche Hornhautreflex unterdrückt werden soll. Dies geschieht durch Anbringen eines Lichtabsorbers in Form eines schwarzen Punkts in der Nähe einer Leuchtfeldblende einer ansonsten bekannten Beleuchtung.

In der US-A-6,011,647 ist ein umschaltbares Beleuchtungssystem für ein ophthalmologisches Operationsmikroskop beschrieben, bei dem zwischen einer Umfeldbeleuchtung und einer optimierten "roter Reflex"-Beleuchtung während der Operation umgeschaltet werden kann. Die Beleuchtungseinrichtung besteht aus Lichtquelle, Kollektor, Leuchtfeldblende, Umlenkspiegel, Feldlinse und Hauptobjektiv. Bei dieser optimierten "roter Reflex"-Beleuchtung wird dann nicht wie bei der Umfeldbeleuchtung die Leuchtfeldblende, sondern die Wendel der Lichtquelle in die Augenpupille als Objektebene abgebildet.

In der EP 1 109 046 A1 ist eine Beleuchtungseinrichtung für ein Operationsmikroskop offenbart, die zwei unabhängig voneinander verschiebbare Reflexionselemente aufweist, mittels derer sowohl die Winkel des einfallenden Lichtes mit der optischen Achse des Mikroskopobjektivs als auch die Intensität der verschiedenen Lichtstrahlen unabhängig voneinander verändert werden können.

Für die Operation am Auge, und hier insbesondere bei Kataraktoperationen, wird ein homogener, heller "roter Reflex" und eine gute Kontrastierung der Phasenobjekte über den gesamten Bereich der Patienten-Augenpupille gefordert.

Die bisher verwendeten Operationsmikroskope erfüllen diese Anforderungen für mehr oder weniger große Bereiche der Augenpupille. Es muss immer ein Kompromiss zwischen den Hauptanforderungen guter, homogener "roter Reflex" und gute Kontrastierung der Phasenobjekte eingegangen werden.

Zumeist wird unter einem kleinen Winkel zur Beobachtung beleuchtet. Dies hat jedoch zur Folge, dass der "rote Reflex" über die Patientenpupille nicht gleichmäßig hell erscheint. Als günstig hat sich bisher ein Beleuchtungswinkel zwischen 2 und 4 Grad bewährt. Bei diesem Winkel erhält man einen guten Kompromiss zwischen guter Kontrastierung und Ausleuchtung der Patientenpupille. Der "rote Reflex" reagiert aber bei dieser Anordnung empfindlich auf ein Verrollen des Patientenauges während der Operation.

Versuche mit einer Koaxial-Beleuchtung führten zwar zu einem guten, homogenen "roten Reflex", aber zu einer schlechten Kontrastierung der Phasenobjekte und haben sich in der Praxis daher bisher nicht bewährt. Dabei war die Beleuchtungsoptik derart angeordnet, dass ein Beleuchtungsspiegel (oder Prisma) zwischen den beiden Strahlengängen des Stereomikroskops lag. Es handelte sich dabei also nicht um eine exakte 0°-Beleuchtung, die genau aus derselben Richtung erfolgt wie die Beobachtung.

In der DE 43 26 761 A1 wird ein Stereomikroskop beschrieben, das eine kontrastreiche Sichtbarmachung transparenter Medien ermöglicht.

In der DE 44 17 273 A1 ist schließlich eine Beleuchtungsvorrichtung für Operationsmikroskope beschrieben, bei der das Beleuchtungsstrahlbündel in wenigstens zwei Beleuchtungsteilstrahlbündel aufgeteilt wird, wobei jedes Beleuchtungsteilstrahlbündel des Beleuchtungsstrahls koaxial zu einem Beobachtungsstrahlbündel verläuft. Dadurch soll der "rote Reflex" verbessert werden.

Ausgehend vom genannten Stand der Technik liegt der vorliegenden Erfindung die Aufgabe zugrunde, ein Operationsmikroskop der Eingangs genannten Art weiterzubilden, um die gewünschte Optimierung weiter zu vervollkommnen. Insbesondere soll ein Operationsmikroskop bereitgestellt werden, mit dem eine optimale Problemlösung der praktischen Anforderungen bezüglich Homogenität des "roten Reflexes" und/oder guter Kontrastierung der Linsenreste beziehungsweise Membranen in der Linsenkapsel realisierbar ist.

Diese Aufgabe wird erfindungsgemäß gelöst durch das Operationsmikroskop mit den Merkmalen gemäß dem unabhängigen Patentanspruch 1. Weitere Vorteile, Merkmale, Details, Aspekte und Effekte der Erfindung ergeben sich aus den Unteransprüchen, der Beschreibung sowie den Zeichnungen.

Der Wesensinhalt der vorliegenden Erfindung liegt daher zunächst in einer neuen Konzeption der Beleuchtungseinrichtung. Die neue Konzeption für die Beleuchtungseinrichtung besteht unter anderem darin, dass diese mindestens zwei, aus einer oder auch mehreren Lichtquellen stammende Strahlbündel erzeugt, wobei die optischen Achsen dieser Strahlbündel koaxial zu den optischen Achsen der Beobachtungsstrahlbündel verlaufen.

Beispielsweise kann eine einzige Lichtquelle vorgesehen sein, die zunächst ein einziges Beleuchtungsstrahlbündel erzeugt. Dieses Beleuchtungsstrahlbündel wird anschließend durch geeignete Mittel, beispielsweise Strahlteiler oder dergleichen, in die gewünschte Anzahl von Beleuchtungsteilstrahlbündeln aufgeteilt. Beispielsweise kann aber auch vorgesehen sein, dass die Beleuchtungseinrichtung zwei oder mehr Lichtquellen aufweist, wobei jede Lichtquelle dann ein Beleuchtungsteilstrahlbündel erzeugt.

Es wird somit eine echte koaxiale Beleuchtung geschaffen. Unter "koaxial" wird dabei generell eine achsnahe Beleuchtung verstanden. Dies schließt sowohl eine Beleuchtung unter exakt Null Grad als auch eine achsnahe Schrägbeleuchtung unter einem sehr kleinen Winkel mit ein. Das könnte als "im Wesentlichen koaxial" bezeichnet werden. Beispiele hierzu werden im weiteren Verlauf der Beschreibung näher erläutert.

Das neue Beleuchtungskonzept aus mindestens zwei koaxialen Beleuchtungsteilstrahlbündeln erzeugt zwei oder mehrere in der Größe variierbare Beleuchtungsspots auf dem Fundus des zu beobachtenden Objekts, eines Auges.

Die Erfindung ist nicht auf eine bestimmte Größe der Beleuchtungsspots beschränkt. Die Beleuchtungsspots weisen eine runde Geometrie auf.

Durch eine saubere, beugungsbegrenzte Abbildung der (Sekundär-)Lichtquelle auf den Fundus erhält man dann zusätzlich zu einem homogenen "roten Reflex" auch eine gute Kontrastierung der Phasenobjekte.

Vorteilhaft können die Durchmesser der Beleuchtungsspots in einem Bereich zwischen 0,5 und 1,5 mm auf dem Fundus des zu beobachtenden Objekts, des Auges, variieren. Natürlich können die Beleuchtungsspots auch einen größeren oder kleineren Durchmesser aufweisen. Vorteilhaft kann der Durchmesser des/der Beleuchtungsspots so ausgebildet sein, dass er auf dem Fundus des zu beobachtenden Auges 1.5 mm, bevorzugt 1.0 mm, weiter bevorzugt 0.5 mm nicht überschreitet.

Die Variation der Beleuchtungsspotdurchmesser wird dabei bedingt durch eine Variation der Beleuchtungsteilstrahlbündel.

Erfindungsgemäss die Beleuchtungsstrahlbündel derart ausgebildet, dass die Größe der Beleuchtungsspots auf dem Fundus des zu beobachtenden Objekts, des Auges, das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

Die Variation der Beleuchtungsspotdurchmesser kann auf unterschiedliche Weise erfolgen. Vorteilhaft geschieht dies unter Zuhilfenahme von Blenden, beispielsweise diskrete Blenden mit verschiedenen Durchmessern oder variable Blenden mit variablen Durchmessern (Irisblenden). Es ist jedoch auch denkbar, hierfür geeignete Displays, beispielsweise LCD-Displays, einzusetzen. Ebenso ist es möglich, die Variation über ein geeignetes Zoom-System zu realisieren. Die letztgenannte Variante hat den weiteren Vorteil, das die Lichtintensität im Beleuchtungsspot zunimmt, wenn der Durchmesser des Beleuchtungsspots verkleinert wird.

Durch die Variation der Beleuchtungsspots auf dem Fundus eines zu untersuchenden Objekts, des Auges, können die Intensität (Helligkeit) und die Homogenität des "roten Reflexes" beeinflusst werden. Je größer der Durchmesser des Beleuchtungsspots gewählt ist, desto homogener und heller ist der "rote Reflex". Je kleiner der Durchmesser des Beleuchtungsspots gewählt ist, desto besser ist die Kontrastierung des "roten Reflexes". Der geeignete Durchmesser eines Beleuchtungsspots ist nunmehr, je nach Bedarf und Anwendungsfall, frei einstellbar.

Mit der Beleuchtungseinrichtung wird eine exakte koaxiale Beleuchtung realisiert, die einen homogenen "roten Reflex" liefert, und die darüber hinaus auch noch gegen ein Verrollen des zu beobachtenden Objekts, eines Patientenauges, unempfindlich ist. Dadurch kann auch eine eventuelle Nachführung der Beleuchtung zur Optimierung des "roten Reflexes" bei verrolltem Auge entfallen, wodurch sich der Aufbau der Beleuchtungseinrichtung beziehungsweise eines entsprechenden Operationsmikroskops vereinfacht.

Besonders vorteilhaft lässt sich die Beleuchtungseinrichtung als - insbesondere duales 0° - Beleuchtungssystem für ein Operationsmikroskop zur Anwendung in der Ophthalmo-Chirurgie einsetzen.

Erfindungsgemäss ist jedes Beleuchtungsteilstrahlbündel derart geführt, dass ein zu beobachtendes Objekt bezüglich jedes Beobachtungsstrahlbündels aus derselben Richtung beleuchtet ist/wird, aus der auch die Beobachtung erfolgt (0°-Beobachtung). Jedes Beleuchtungsteilstrahlbündel wird derart geführt, dass für den linken und rechten Beobachtungsstrahlengang des (Stereo-)Operationsmikroskops das zu beobachtende Objekt, ein Auge, aus derselben Richtung beleuchtet wird, aus der auch die Beobachtung erfolgt. Es liegt somit eine exakte 0°-Beleuchtung für jeden Beobachtungsstrahlengang vor.

Gemäß einer anderen erfindungsgemässen Ausgestaltung ist jedes Beleuchtungsteilstrahlbündel derart geführt, dass ein zu beobachtendes Objekt bezüglich jedes Beobachtungsstrahlbündels in einem Winkel von kleiner/gleich 2 Grad, vorzugsweise kleiner/gleich 1 Grad schräg beleuchtet ist/wird (achsnahe Schrägbeleuchtung). Das zu untersuchende Objekt wird somit in einem kleinen Winkel zur Beobachtung beleuchtet.

Durch eine saubere, beugungsbegrenzte Strahlführung für die Beleuchtung und kleine Beleuchtungsspots auf dem Fundus eines Patientenauges erhält man einen optimalen "roten Reflex" bei gleichzeitig guter Kontrastierung. Ferner reagiert diese Beleuchtungseinrichtung sehr unkritisch auf ein Verrollen des Patientenauges während der Operation.

Bezüglich der erfindungsgemäßen Ausgestaltung der Beleuchtungseinrichtung wird ebenfalls auf die vorstehenden Ausführungen zum ersten Erfindungsaspekt Bezug genommen und hiermit verwiesen.

Vorteilhaft kann vorgesehen sein, dass die Größe des/der Beleuchtungsspots auf dem Fundus des zu beobachtenden Objekts das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

Erfindungsgemäß wird eine Beleuchtungseinrichtung für eine zwei oder mehr Beobachtungsstrahlengänge mit jeweils einem Beobachtungsstrahlbündel aufweisende Beobachtungseinrichtung, bereitgestellt, aufweisend zumindest eine Lichtquelle zum Erzeugen von zwei Beleuchtungsstrahlbündeln zum Beleuchten eines zu beobachtenden Auges. Erfindungsgemäß ist vorgesehen, dass
die zwei Beleuchtungsstrahlbündel wenigstens zwei Beleuchtungsspots auf dem Fundus des zu beobachtenden Augesbildenund dass die Größe der Beleuchtungsspots auf dem Fundus des zu beobachtenden Auges das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

Die zwei Beleuchtungsstrahlbündel verlaufen koaxial zu den entsprechenden Beobachtungsstrahlbündeln.

In weiterer Ausgestaltung ist vorteilhaft vorgesehen, dass die Entfernung der Mitte des Beleuchtungsspots von der Mitte der Querschnittsfläche des Beobachtungsstrahlbündels auf dem Fundus das 0.8-fache, bevorzugt das 0.5-fache, weiter bevorzugt das 0.2-fache, besonders bevorzugt das 0.05-fache des Radius der Querschnittsfläche des Beobachtungsstrahlbündels auf dem Fundus beträgt.

Durch die Beleuchtungseinrichtung kann insbesondere erreicht werden, dass sich die optimale Größe des Beleuchtungsspots nach der Fehlsichtigkeit des Patienten und der Vergrößerung des Operationsmikroskops richtet. Die wird beispielsweise durch die Relativangaben von Beleuchtungsspotgröße zu Querschnittsfläche der Beobachtungsstrahlenkegel auf dem Fundus erreicht. Es werden die wesentlichen Merkmale für einen optimalen Rotreflex verwirklicht, nämlich kleine Spotgröße für guten Kontrast sowie die Lage des Beleuchtungsspots auf dem Fundus.

Eine vorteilhafte Ausgestaltung der Erfindung sieht eine Beleuchtungseinrichtung für eine zwei oder mehr Beobachtungsstrahlengänge mit jeweils einem Beobachtungsstrahlbündel aufweisende Beobachtungseinrichtung, vor, aufweisend zumindest eine Lichtquelle zum Erzeugen von zwei Beleuchtungsstrahlbündeln
zum Beleuchten
eines zu beobachtenden Auges, wobei die Größe 'der Beleuchtungsspots auf dem Fundus des zu beobachtenden Auges das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet, und wobei die Entfernung der Mitte des Beleuchtungsspots von der Mitte der Querschnittsfläche des Beobachtungsstrahlbündels auf dem Fundus das 0.8-fache, bevorzugt das 0.5-fache, weiter bevorzugt das 0.2-fache, besonders bevorzugt das 0.05-fache des Radius der Querschnittsfläche des Beobachtungsstrahlbündels auf dem Fundus beträgt.

Vorteilhaft kann der Durchmesser der Beleuchtungsspots in einem Bereich zwischen 0,5 und 1,5 mm auf dem Fundus des zu beobachtenden Objekts variierbar sein. Vorteilhaft kann der Durchmesser der Beleuchtungsspots so ausgebildet sein, dass er auf dem Fundus des zu beobachtenden Objekts 1.5 mm, bevorzugt 1.0 mm, weiter bevorzugt 0.5 mm nicht überschreitet.

Vorteilhaft kann die Beleuchtungseinrichtung wenigstens ein Objektivelement aufweisen. Das Objektivelement kann dabei ebenfalls als Objektivelement des Operationsmikroskops, insbesondere als dessen Hauptobjektiv, ausgebildet sein. Dies ist jedoch nicht zwingend erforderlich.

Weiterhin können in der Beleuchtungseinrichtung verschiedene optische Elemente vorgesehen sein, die zwischen der wenigstens einen Lichtquelle und dem wenigstens einen Objektivelement angeordnet sind.

In vorteilhafter Ausgestaltung sind Mittel vorgesehen, um jeweils ein Beobachtungsstrahlbündel und ein Beleuchtungsteilstrahlbündel beziehungsweise das Beleuchtungsstrahlbündel zu überlagern. Diese Mittel können auf unterschiedlichste Weise ausgestaltet und an unterschiedlichsten Orten angeordnet sein. Nachfolgend werden hierzu einige nicht ausschließliche Beispiele erläutert.

Beispielsweise kann vorgesehen sein, dass die Mittel zum Überlagern derart angeordnet sind, dass eine Überlagerung von Beobachtungsstrahlbündel und Beleuchtungsteilstrahlbündel beziehungsweise Beleuchtungsstrahlbündel oberhalb des Objektivelements erfolgt. Die Überlagerung von Beobachtungsstrahlbündel und Beleuchtungsteilstrahlbündel beziehungsweise Beleuchtungsstrahlbündel kann beispielsweise im parallelen Strahlengang über dem Hauptobjektiv erfolgen.

Beispielsweise kann auch vorgesehen sein, dass die Mittel zum Überlagern derart angeordnet sind, dass eine Überlagerung von Beobachtungsstrahlbündel und Beleuchtungsteilstrahlbündel beziehungsweise Beleuchtungsstrahlbündel unterhalb des Objektivelements erfolgt. Es besteht somit auch die Möglichkeit, die Beleuchtungsteilstrahlbündel beziehungsweise das Beleuchtungsstrahlbündel und die Beobachtungsstrahlbündel unterhalb des Hauptobjektivs zu überlagern. In diesem Fall ist es vorteilhaft, wenn die Beleuchtungsteilstrahlbündel entsprechend der Hauptobjektivbrennweite geneigt werden.

Vorteilhaft kann insbesondere im letztgenannten Fall vorgesehen sein, dass das Objektivelement als so genannte Varioskopoptik ausgebildet ist. Bei einer Varioskopoptik handelt es sich generell um eine Optik mit wenigstens zwei durch einen Abstand getrennten optischen Elementen, wobei durch Variation dieses Abstands der freie Arbeitsabstand zwischen Objektiv und Objektebene variiert werden kann. Eine solche Varioskopoptik an sich ist bereits aus dem Stand der Technik bekannt. In dem zuvor beschriebenen Fall mit Überlagerung der Strahlbündel unterhalb des Objektivelements ist es bei Verwendung einer Varioskopoptik vorteilhaft, wenn die Beleuchtungsteilstrahlbündel entsprechend dem freien Arbeitsabstand nachgeführt werden.

Wie oben schon ausgeführt wurde, ist die Erfindung nicht auf bestimmte Ausgestaltungstypen von "Überlagerungsmitteln" beschränkt. Beispielsweise können die Mittel zum Überlagern wenigstens ein optisches Element in Form eines Prismas und/oder einer Strahlteilerplatte und/oder eines Spiegels, etwa eines teildurchlässigen Spiegels und/oder eines durchbohrten Spiegels, aufweisen. Natürlich können die Mittel auch anders ausgestaltet sein, so dass die Erfindung nicht auf die genannten Beispiele beschränkt ist.

Vorteilhaft kann wenigstens eine Vorrichtung zum Verändern des Bündelquerschnitts des wenigstens eines Beleuchtungsstrahls und/oder wenigstens eines Beleuchtungsteilstrahlbündels vorgesehen ist. Dabei ist die Erfindung nicht auf bestimmte Ausführungsformen der Vorrichtung beschränkt. Die Vorrichtung kann beispielsweise als Blende, insbesondere Irisblende oder diskrete Blende, als LCD (Liquid Crystal Display) Display, als DMD (Digital Mirror Device), als LCOS (Liquid Crystal On Silicon), als FLCOS (Ferroelectric Liquid Crystal On Silicon) oder dergleichen ausgebildet sein. Durch den Einbau einer entsprechenden Vorrichtung in der Beleuchtungseinrichtung, etwa im Beleuchtungsstrahl, ist es möglich, den Leuchtfleck auf der Oberfläche des zu beobachtenden Objekts, beispielsweise auf dem Fundus des Patientenauges, zu variieren. Ein kleiner Lichtfleck liefert einen besseren Kontrast. Bei Kataraktoperationen kann der Fall auftreten, dass insbesondere bei dichten Katarakten der "rote Reflex" zu dunkel erscheinen kann. Dann ist es von Vorteil, den Leuchtfleck und damit die Helligkeit zu vergrößern. Die Intensität der Strahlung auf der Retina wird dadurch nicht vergrößert. Negative Auswirkungen auf den Kontrast sind nicht zu erwarten, da bei einem sehr dichten Katarakt der Leuchtfleck ohnehin aufgestreut wird.

Ein weiterer Vorteil der Beleuchtungseinrichtung besteht darin, dass auf der Hornhaut-Vorderfläche des Patientenauges nur ein Hornhautreflex sichtbar wird, da sich die Beleuchtungsteilstrahlbündel an dieser Stelle annähernd überdecken.

In einer vorteilhaften Ausgestaltung kann vorgesehen sein, dass zwei oder mehr Lichtquellen vorgesehen sind und dass mittels jeder Lichtquelle ein Beleuchtungsteilstrahlbündel erzeugt wird. Es können somit unabhängige Lichtquellen verwendet werden, wobei jede Lichtquelle ein eigenes Beleuchtungsteilstrahlbündel erzeugt.

In einer anderen vorteilhaften Ausgestaltung kann vorgesehen sein, dass eine einzige Lichtquelle vorgesehen ist und dass Mittel zum Aufteilen des Beleuchtungsstrahlbündels der Lichtquelle in die zwei oder mehr Beleuchtungsteilstrahlbündel vorgesehen sind. Hierbei kann es sich um geeignete Strahlteiler in Form von Prismen, teildurchlässigen Spiegeln und dergleichen handeln.

Die vorliegende Erfindung ist nicht auf die Verwendung bestimmter Lichtquellen beschränkt. Nachfolgend werden hierzu einige nicht ausschließliche, vorteilhafte Beispiele genannt. Beispielsweise kann die wenigstens eine Lichtquelle als Lampe, insbesondere als Halogenlampe oder Xenonlampe, als Laser, als nicht thermischer Strahler, als Lichtleiter, insbesondere als Faserlichtleiterbündel, als wenigstens eine LED (Licht emittierende Diode), als wenigstens eine OLED (organische Licht emittierende Diode) oder dergleichen ausgebildet sein. Natürlich sind auch Kombinationen verschiedener Lichtquellen möglich.

Vorteilhaft ist die Lichtquelle aus einer Anordnung von einer oder mehreren einzeln oder bereichsweise schaltbaren Kleinstlichtquelle(n) gebildet. Die Beleuchtungseinrichtung ist dabei so ausgestaltet, dass sie bezüglich der von ihr erzeugten Leuchtfeldgeometrie einfach variiert werden kann. Dabei werden die Kleinstlichtquellen - insbesondere elektronisch - von außen, vorzugsweise von einer Steuereinrichtung, angesteuert. Ein weiteres Merkmal sieht vor, dass die Kleinstlichtquellen zumindest bereichsweise ansteuerbar sind, um variable Beleuchtungsgeometrien einstellen zu können. Dies ist insbesondere bei der Erzeugung von ringförmigen Beleuchtungsteilstrahlbündeln von Vorteil. Dabei ist die Erfindung nicht auf bestimmte Größen und/oder Formen von Bereichen beschränkt. Im einfachsten Fall kann ein einziger Punkt in solch einer Weise ansteuerbar sein. Insbesondere dann, wenn die Leuchtquelle aus einer Matrix bestehend aus einzelnen Kleinstlichtquellen gebildet ist, kann eine oder können mehrere Kleinstlichtquellen einzeln oder in Gruppen ansteuerbar sein, wobei im letztgenannten Fall einzelne Kleinstlichtquellen zu einem Bereich zusammengefasst werden können. Auch diesbezüglich ist die Erfindung nicht auf konkrete Ausgestaltungsformen beschränkt.

Vorteilhaft kann die Lichtquelle aus einer Anordnung von einer oder mehreren Leuchtdiode(n) (LED), insbesondere organischen Leuchtdiode(n) (OLED), gebildet sein. Organische Leuchtdioden sind ursprünglich als Mikrodisplays entwickelt worden. Anders als LCDs, die eine Hinterleuchtung benötigen, leuchten OLEDs selber als Lambertstrahler (Flächenemitter).

Als strukturierte Beleuchtungsquelle bieten OLEDs eine gute Lichteffizienz und kleine Strukturen ohne dunkle Zwischenräume. Entsprechend einer gewünschten Beleuchtungsgeometrie können einzelne der Kleinstlichtquellen angeschaltet werden und andere ausgeschaltet bleiben. Gegenüber LEDs ist bei OLEDs der Füllfaktor höher was bedeutet, dass eine höhere Packungsdichte realisierbar ist. Die Verwendung eines Displays aus LEDs oder OLEDs ermöglicht ein programmierbares, und beispielsweise auch automatisierbares Schalten unterschiedlicher Beleuchtungsmodi, ohne dass mechanische Komponenten, wie etwa Phasenkontrastringe, Filter, Abschwächer und dergleichen bewegt werden müssten. Besonders geeignet sind beispielsweise weiße OLEDs, deren Spektrum durch eine Mischung von organischen Molekülen bestimmt wird.

Zusammenfassend weist das wie vorstehend beschriebene Operationsmikroskop eine ganze Reihe von Vorteilen auf. Durch eine koaxiale Beleuchtung, insbesondere durch eine "echte" 0°-Beleuchtung, lässt sich ein sehr homogener und heller "roter Reflex" erzeugen. Der "rote Reflex" reagiert auf ein Verkippen des zu beobachtenden Objekts, eines Patientenauges, sehr unempfindlich. Das heißt, auf eine Nachführung bezüglich Winkeln kann verzichtet werden. Über die Integration einer Vorrichtung zum Verändern des Strahlbündelquerschnitts, beispielsweise einer (Doppel-)Irisblende kann die Helligkeit des "roten Reflexes" und der Kontrast von Phasenstrukturen an die Behandlungssituation angepasst und optimiert werden. Durch die Verringerung des Irisblendendurchmessers wird der Kontrast verbessert, allerdings nimmt auch die Helligkeit ab.

Gemäß noch einem weiteren Aspekt wird ein Operationsmikroskop bereitgestellt, mit
zwei oder mehr stereoskopischen Beobachtungsstrahlengängen mit jeweils einem Beobachtungsstrahlbündel und mit einer Beleuchtungseinrichtung, aufweisend zumindest eine Lichtquelle zum Erzeugen von zwei Beleuchtungsstrahlbündeln
zum Beleuchten eines
Auges. Dieses Operationsmikroskop ist dadurch gekennzeichnet, dass Beleuchtungsstrahlbündel beugungsbegrenzt abgebildet wird und dass die Beleuchtungsstrahlbündel zwei in der Größe variierbare Beleuchtungsspots auf dem Fundus des zu beobachtenden Objekts bilden.

Erfindungsgemäß wird ein Operationsmikroskop bereitgestellt, mit zwei oder mehr stereoskopischen Beobachtungsstrahlengängen mit jeweils einem Beobachtungsstrahlbündel und mit einer Beleuchtungseinrichtung, aufweisend zumindest eine Lichtquelle zum Erzeugen von zwei Beleuchtungsstrahlbündeln
zum Beleuchten eines zu beobachtenden Auges. Dieses Operationsmikroskop ist erfindungsgemäß dadurch gekennzeichnet, dass die Größe der Beleuchtungsspots auf dem Fundus das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

Vorteilhaft kann der Durchmesser der Beleuchtungsspots in einem Bereich zwischen 0,5 und 1,5 mm auf dem Fundus des zu beobachtenden Auges variieren. Vorteilhaft kann der Durchmesser der Beleuchtungsspots so ausgebildet sein, dass er auf dem Fundus des zu beobachtenden Objekts 1.5 mm, bevorzugt 1.0 mm, weiter bevorzugt 0.5 mm nicht überschreitet.

Vorteilhaft ist die Beleuchtungseinrichtung in der wie vorstehend beschriebenen Weise ausgebildet, so dass auf die entsprechenden Ausführungen Bezug genommen und verwiesen wird.

Das Operationsmikroskop kann beispielsweise ein Hauptobjektivelement aufweisen, welches identisch mit einem Objektivelement der Beleuchtungseinrichtung ist. Weiterhin können Mittel vorgesehen sein, um jeweils ein Beobachtungsstrahlbündel und ein Beleuchtungsteilstrahlbündel beziehungsweise Beleuchtungsstrahlbündel zu überlagern. Die Mittel zum Überlagern können derart angeordnet sein, dass eine Überlagerung von Beobachtungsstrahlbündel und Beleuchtungsteilstrahlbündel beziehungsweise Beleuchtungsstrahlbündel oberhalb des Hauptobjektivelements erfolgt.

In anderer Ausgestaltung kann vorgesehen sein, dass das Operationsmikroskop ein Hauptobjektivelement aufweist, welches identisch mit einem Objektivelement der Beleuchtungseinrichtung ist, dass Mittel vorgesehen sind, um jeweils ein Beobachtungsstrahlbündel und ein Beleuchtungsteilstrahlbündel beziehungsweise Beleuchtungsstrahlbündel zu überlagern und dass die Mittel zum Überlagern derart angeordnet sind, dass eine Überlagerung von Beobachtungsstrahlbündel und Beleuchtungsteilstrahlbündel beziehungsweise Beleuchtungsstrahlbündel unterhalb des Hauptobjektivelements erfolgt.

Für den letztgenannten Fall kann vorteilhaft vorgesehen sein, dass das Hauptobjektivelement als so genannte Varioskopoptik ausgebildet ist. Zu der Ausgestaltung und Funktionsweise der Varioskopoptik wird auf die entsprechenden Ausführungen weiter oben im Zusammenhang mit der erfindungsgemäßen Beleuchtungseinrichtung verwiesen.

Vorteilhaft kann das Operationsmikroskop als stereoskopische Beobachtungseinrichtung, insbesondere als Stereomikroskop, ausgebildet sein. Das optische System eines Operationsmikroskops besteht grundsätzlich aus mehreren Bauelementen, wie dem Tubus, dem Mikroskop-Grundkörper, usw. Zusätzlich ist es bei vielen Operationsmikroskopen möglich, unterschiedliche Zusatzmodule, wie zum Beispiel einen Mitbeobachtertubus für einen assistierenden Beobachter, eine Videokamera zur Dokumentation oder dergleichen anzuschließen.

Innerhalb des Mikroskop-Grundkörpers lassen sich wiederum mehrere Baugruppen zusammenfassen, wie beispielsweise eine Beleuchtungseinrichtung, eine Vergrößerungseinrichtung, das Hauptobjektiv oder dergleichen. Die charakteristische Größe beim Hauptobjektiv ist seine Brennweite, die den Arbeitsabstand vom Operationsmikroskop zum Operationsfeld festlegt und auch Einfluss auf die Gesamtvergrößerung des Mikroskops hat.

Vorzugsweise kann in dem wenigstens einen Beobachtungsstrahlengang ein Vergrößerungssystem vorgesehen sein. Hierbei kann es sich beispielsweise um einen Vergrößerungswechsler handeln, mit dem sich unterschiedliche Vergrößerungen einstellen lassen. In vielen Anwendungsfällen ist ein Vergrößerungswechsel in Stufen völlig ausreichend. Es ist jedoch auch möglich, als Vergrößerungssystem auch pankratische Vergrößerungssysteme zu verwenden, mittels derer eine stufenlose Vergrößerung (Zoomsystem) möglich ist.

Dabei kann vorteilhaft vorgesehen sein, dass die weiter oben bereits beschriebene Gerätepupille der Beobachtungseinrichtung in dem Vergrößerungssystem angesiedelt ist.

Weiterhin können in dem wenigstens einen Beobachtungsstrahlengang ein Tubuselement und ein Okularelement vorgesehen sein. Die Aufgabe eines Okularelements ist generell die Nachvergrößerung des im Tubus entstehenden Zwischenbilds, sowie möglicherweise der Ausgleich eventueller Fehlsichtigkeiten des Nutzers eines solchen Mikroskops.

Vorteilhaft ist weiterhin vorgesehen, dass die Objektebene des zu untersuchenden Objekts, des Auges, im vorderen Brennpunkt des Hauptobjektivs ausgebildet ist. Dadurch wird erreicht, dass das zu untersuchende Objekt durch das Hauptobjektiv nach Unendlich abgebildet wird.

Vorteilhafterweise ist das Operationsmikroskop als stereoskopisches Operationsmikroskop, insbesondere als Stereomikroskop, ausgebildet. In diesem Fall verfügt die Beobachtungseinrichtung über zwei parallel verlaufende Beobachtungsstrahlengänge.

Bei dem Operationsmikroskop kann es sich gemäß einer bevorzugten Ausführungsform um ein Stereomikroskop nach dem Teleskopprinzip handeln, das im Wesentlichen aus den drei optischen Teilkomponenten, nämlich Hauptobjektiv (afokales) Zoomsystem sowie binokulares Fernrohr aus Tubus und Okularen, besteht.

Zwischen den einzelnen Teilkomponenten des Operationsmikroskops verlaufen die Beobachtungsstrahlenbündel vorzugsweise parallel, sodass die einzelnen Teilkomponenten modular austauschbar und kombinierbar sind.

In bevorzugter Weise kann eine wie vorstehend beschriebene Beleuchtungseinrichtung in einem Operationsmikroskop, insbesondere in einer ophthalmologischen Beobachtungseinrichtung, vorzugsweise in einem für die Kataraktextraktion ausgebildeten Operationsmikroskop, verwendet werden. Ebenso kann ein wie vorstehend beschriebenes erfindungsgemäßes Operationsmikroskop vorteilhaft als ophthalmologische Beobachtungseinrichtung, vorzugsweise als ein für die Kataraktextraktion ausgebildetes Operationsmikroskop, verwendet werden.

Gemäß der vorliegenden Erfindung sind insbesondere die prinzipiellen Grundanforderungen für ein optimiertes Beleuchtungssystem in der Kataraktchirurgie festgelegt worden, nämlich eine koaxiale Beleuchtung für die Homogenität des roten Reflexes eine stigmatische, beugungsbegrenzte Abbildung der wohldefinierten Beleuchtungsspots für die gute Kontrastierung des Roten Reflexes.

Für die Erzeugung der koaxialen Beleuchtungsstrahlenbündel wird beispielsweise ein Prismensystem vorgeschlagen. Die Größe der Beleuchtungsspots auf dem Fundus kann durch geeignete Anpassung von Aperturblenden beim Prismensystem gezielt eingestellt werden.

Darüber hinaus gestattet das erfindungsgemäße Beleuchtungssystem eine einfache Umschaltung zwischen der optimierten Red Reflex - Beleuchtung und der für die praktische Anwendung unabdingbaren Umfeldbeleuchtung zur vollständigen Ausleuchtung des maximalen Sehfelds bei der stereoskopischen Beobachtung.

Die Erfindung wird nun anhand von Ausführungsbeispielen unter Bezugnahme auf die beiliegenden Zeichnungen näher erläutert. Es zeigen
- Figur 1: in schematischer Darstellung eine mögliche Anordnung zur Erzeugung einer 0°-Beleuchtung, bei gleichzeitig optimalem "roten Reflex" und guter Kontrastierung;
- Figur 2: in schematischer Darstellung den Aufbau eines optischen Systems für die Rotreflex-Beleuchtung;
- Figur 3: in schematischer Darstellung den Aufbau eines optischen Systems für die Umfeldbeleuchtung; und
- Figur 4: in schematischer Darstellung den Aufbau einer vorteilhaften Aperturblende, wie sie in den Beleuchtungseinrichtung gemäß Figur 2 eingesetzt ist.

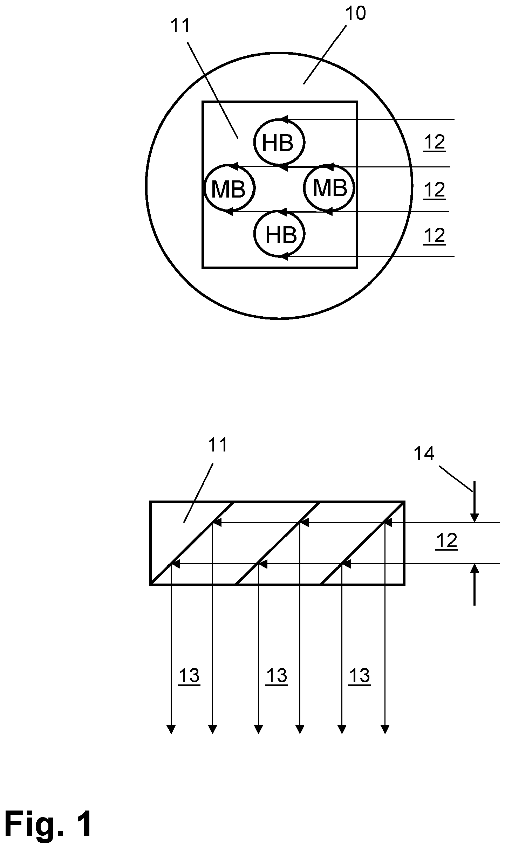

In Figur 1 sind Teile einer Beleuchtungseinrichtung dargestellt, die in einem Operationsmikroskop eingesetzt ist. Bei dem Operationsmikroskop handelt es sich um ein Stereo-Operationsmikroskop zum Einsatz in der Ophthalmo-Chirurgie, beispielsweise zur Durchführung von Kataraktoperationen. Mittels der Beleuchtungseinrichtung wird ein sehr gleichmäßiger, heller "roter Reflex" dadurch erzielt, dass das Beleuchtungsstrahlbündel 12 in mehrere Beleuchtungsteilstrahlbündel 13 aufgeteilt wird. Dies geschieht durch Mittel 11 zum Aufteilen des Beleuchtungsstrahlbündels, welche hierfür über eine geeignete Spiegel/Prismenanordnung verfügen können. Die Beleuchtungsteilstrahlbündel 13 sind dabei so geführt, dass bezüglich des linken und rechten Beobachtungsstrahlengangs des Operationsmikroskops das zu beobachtende Objekt, im vorliegenden Fall ein Patientenauge, aus derselben Richtung beleuchtet wird, aus der auch die Beobachtung erfolgt (0°-Beleuchtung).

Wie dem linken Teil der Figur zu entnehmen ist, sind bei dem dargestellten Beispiel Beobachtungssstrahlengänge sowohl für einen Hauptbeobachter (HB) als auch für einen Mitbeobachter (MB) vorgesehen. Die Mittel 11 zur Aufteilung des Beleuchtungsstrahlengangs können beispielsweise im Bereich eines Objektivelements 10 angeordnet sein, bei dem es sich beispielsweise auch um das Hauptobjektiv der Beobachtungseinrichtung handeln kann.

Durch eine saubere, beugungsbegrenzte Strahlführung für die Beleuchtung und kleine Beleuchtungsspots auf dem Fundus des Patientenauges (Durchmesser etwa 0,5 bis 1,5mm) erhält man mit dieser Anordnung einen optimalen "roten Reflex" bei gleichzeitig guter Kontrastierung. Ferner reagiert diese Beleuchtungsanordnung sehr unkritisch auf ein Verrollen des Patientenauges während der Operation.

Die Überlagerung von Beobachtungsstrahlbündel und Beleuchtungsteilstrahlbündel 13 kann beispielsweise im parallelen Strahlengang über dem Objektivelement 10 (dem Hauptobjektiv) durch die Mittel 11 erfolgen, bei denen es sich vorteilhaft um teildurchlässige Spiegel oder Prismen handelt.

Ferner ist in der Beleuchtungseinrichtung eine Vorrichtung 14 zum Verändern der Strahlbündelquerschnitte in Form einer Irisblende vorgesehen. Dadurch kann im Beleuchtungsstrahl 12 der Leuchtfleck auf dem Fundus des Patientenauges variiert werden.

Im vorliegenden Beispiel wird das Beleuchtungsstrahlbündel 12 von einer einzigen Lichtquelle (nicht dargestellt) erzeugt und über die Mittel 11 auf mehrere Beleuchtungsteilstrahlbündel 13 aufgeteilt. Es ist jedoch auch denkbar, mehrere voneinander unabhängige Lichtquellen zu verwenden, wobei jede Lichtquelle jeweils mindestens ein Beleuchtungsteilstrahlbündel 13 erzeugt.

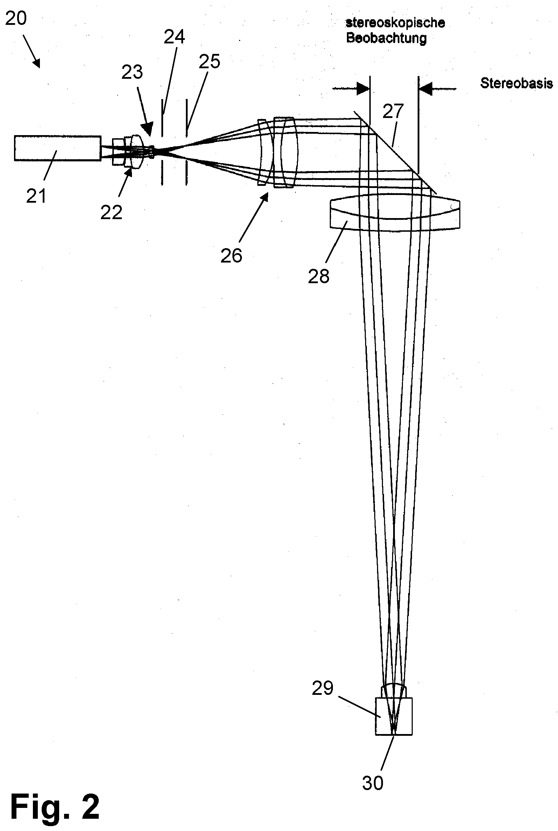

Der Aufbau des optischen Systems für die Rotreflex - Beleuchtung ist in Figur 2 skizziert. Von einer Lichtquelle aus betrachtet werden in der dargestellten Beleuchtungseinrichtung 20 folgende optischen Komponenten verwendet: ein Lichtleiter 21, ein Kollektor 22, eine Plankonvexlinse 23, eine Leuchtfeldblende 24, eine Aperturblende (Lochblende) 25, eine optische Komponente 26, beispielsweise eine Teilkomponente aus Kittglied und Meniskus, ein Umlenkelement 27, beispielsweise in Form eines Teilerspiegels sowie ein Objektivelement 28, beispielsweise in Form eines Hauptobjektivs. Beleuchtet werden soll ein Auge 29 mit Fundus 30.

Vom Faserende des Lichtleiters 21 wird mit einem Kollektor 22 und einer Plankonvexlinse 23 ein reelles Zwischenbild erzeugt. Am Ort dieses Zwischenbilds kann eine Aperturblende 25 z.B. in Form einer Lochblende angebracht werden. Dieses reelle Zwischenbild liegt in der vorderen Brennebene einer zweigliedrigen Teiloptik bestehend aus dem Hauptobjektiv 28 und der Teilkomponente 26 aus Kittglied mit Meniskuslinse. Diese Teiloptik bildet dann ein weiteres virtuelles Zwischenbild im Unendlichen, so dass vom Auge 29 aus betrachtet das Faserende des Lichtleiters 21 im Fernpunkt liegt. Infolgedessen wird dann bei einem rechtsichtigen Auge das Faserende des Lichtleiters 21 als Beleuchtungsspot auf den Fundus 30 abgebildet.

Die wirksame Leuchtfläche des Faserendes vom Lichtleiter 21 kann beispielsweise 4.8 mm betragen. Der Durchmesser des Zwischenbilds in der Aperturblende 25 beträgt dann 5.8 mm. Für die Größe des Beleuchtungsspots auf dem Fundus 30 erhält man in dem genannten Beispiel einen Durchmesser von 1.5 mm.

Mittels Lochblenden wohldefinierter Lage können in der Zwischenbildebene (Aperturblende) die für die gute Homogenität des Roten Reflexes erforderlichen zu den stereoskopischen Beobachtungsachsen koaxialen Strahlenbüschel im Abstand der Stereobasis der stereoskopischen Beobachtungsachsen erzeugt werden. Die Lochblenden werden bezüglich Lage, also Lateralversatz zur optischen Achse, und Größe, also Durchmesser der Lochblenden, mit demselben Abbildungsmaßstab wie das reelle Zwischenbild des Faserendes, nämlich 5.8 : 1.5 = 3.9 : 1, verkleinert auf den Fundus abgebildet. Die Grösse des Durchmessers der Lochblenden bestimmt dann die Größe der Beleuchtungsspots auf dem Fundus und somit in entscheidender Weise die gute Kontrastierung des Roten Reflexes.

Zwischen der Plankonvexlinse 23 und dem reellen Zwischenbild des Faserendes (Aperturblende) befindet sich die Leuchtfeldblende 24. Diese Leuchtfeldblende 24 dient zur Begrenzung des ausgeleuchteten Sehfelds.

Die Leuchtfeldblende 24 liegt im vorderen Brennpunkt der Teilkomponente 26 aus Kittglied und Meniskuslinse. Die Leuchtfeldblende 24 wird also durch die Teilkomponente 26 zunächst virtuell nach Unendlich und schließlich mit dem Hauptobjektiv 28 auf die in der vorderen Brennebene des Hauptobjektivs sich befindliche Objektebene abgebildet.

Der Durchmesser der Leuchtfeldblende 24 beträgt beispielsweise 2.5 mm. Dies führt zu einem ausgeleuchteten Sehfeld in der Objektebene von 10 mm. Somit beträgt der Abbildungsmaßstab für die Leuchtfeldabbildung 1 : 4.

In der Tabelle 1 sind die optischen Systemdaten für die Rotreflex - Beleuchtung aufgelistet:

### Systemdaten für Red Reflex - Beleuchtungssystem

**Tabelle 1**

| **Nr.** | **Radius** (mm) | **Dicke** (mm) **Lichtleiter** | **Medium** | **freier Durchmesser** (mm) |
|---|---|---|---|---|
| 1 | -49.759 | 4.7 | Luft | 8.5 |
| 2 | -17.655 | 5.0 | NSK2 | 10.8 |
| 3 | -37.047 | 0.1 | Luft | 11.0 |
| 4 | 26.227 | 2.0 | NSF6 | 12.2 |
| 5 | -12.589 | 5.5 | NSK2 | 13.6 |
| 6 | 6.6355 | 2.0 | Luft | 5.0 |
| 7 | Plan | 2.0 | NSK2 | 5.0 |
| 8 | Plan | 1.9 | Luft | **Blende** |
| 9 | Plan | 13.1 | Luft | **Blende** |
| 10 | -58.294 | 29.2 | Luft | 26.0 |
| 11 | -28.387 | 5.0 | NSK2 | 27.0 |
| 12 | 392.42 | 0.1 | Luft | 28.0 |
| 13 | 45.316 | 3.0 | NSF6 | 29.0 |
| 14 | -55.033 | 7.0 | NSK2 | 29.0 |
| 15 | Plan | 40.0 | Luft | Spiegel |
| 16 | 120.57 | 17.0 | Luft | 53.0 |
| 17 | -79.719 | 10.5 | NFK51 | 53.0 |
| 18 | -244.06 | 5.1 | NBAF4 | 53.0 |
| 188.3 | | Luft | | |

| **Nr.** | **Radius** (mm) | **Dicke** (mm) | **Medium** | |
|---|---|---|---|---|
| 19 | 8.0 | 6.0 | BAK4 | |
| 20 | Plan | 15.4 | BK7 | **Modellauge** |
| | | **Fundus** | | |

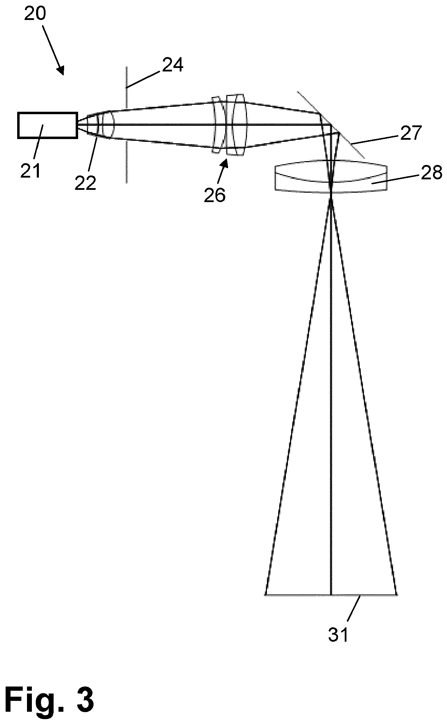

Der Aufbau des optischen Systems für die Umfeldbeleuchtung ist in Figur 3 skizziert.

Ein wesentlicher Gedanke besteht darin, dass die Umfeld - Beleuchtung ohne zusätzliche optische Komponenten durch einen einfachen Umschaltvorgang aus der Rotreflex - Beleuchtung abgeleitet werden kann.

Die für die Umfeld - Beleuchtung notwendigen optischen Komponenten sind also bis auf die Größe der Leuchtfeldblende identisch mit den optischen Komponenten der Rotreflex Beleuchtung gemäß Figur 2, nämlich: Lichtleiter 21, Kollektor 22, Leuchtfeldblende 24, optische Komponente 26, beispielsweise in Form einer Teilkomponente aus Kittglied und Meniskus, Umlenkelement 27, beispielsweise in Form eines Teilerspiegels, sowie Objektivelement 28, beispielsweise in Form eines Hauptobjektivs.

Bei der Umschaltung von der Rotreflex - Beleuchtung zur Umfeldbeleuchtung wird die Plankonvexlinse und die Lochblende (siehe Figur 2) herausgeschwenkt. Des Weiteren wird eine kleine Leuchtfeldblende durch eine große Leuchtfeldblende 24 ersetzt.

Mit dem Kollektor 22 wird nun die Leuchtfeldblende 24 vollständig ausgeleuchtet. Die Leuchtfeldblende 24 sitzt ja wie bei der Rotreflex Beleuchtung unverändert in der vorderen Brennebene der Teiloptik 26 aus Kittglied und Meniskuslinse. Die Leuchtfeldblende 24 wird also virtuell nach Unendlich abgebildet, so dass das Bild der Leuchtfeldblende 24 wie bei der Rotreflex - Beleuchtung durch die Abbildung mit dem Hauptobjektiv 28 wiederum in der vorderen Fokusebene des Hauptobjektivs 28 und somit in der Objektebene der Beobachtung liegt.

Der Durchmesser der Leuchtfeldblende 24 beträgt beispielsweise 14 mm. Damit kann man eine Ausleuchtung des maximalen Sehfelds 31 in der Objektebene von ca. 62 mm erreichen. Die Vergrößerung des Abbildungsmaßstabs gegenüber der Rotreflex Beleuchtung ist durch die Verzeichnung der Leuchtfeldabbildung erklärbar.

Der optische Aufbau für die hier vorgeschlagene Rotreflex Beleuchtung ermöglicht einen unabhängigen Eingriff in die Strahlengänge für die Pupillen- und Leuchtfeldabbildung. So kann beispielsweise die Lichtausbeute optimal an die Leuchtfeldgröße angepasst und die Größe der Beleuchtungsspots auf dem Fundus durch gezielten Eingriff in der Aperturblende angepasst werden.

Bei der Umfeld - Beleuchtung reduziert sich die optische Problemstellung auf die Abbildung der optimal ausgeleuchteten Leuchtfeldblende.

Für die Pupillenabbildung ergibt sich aufgrund des optischen Aufbaus zwangsläufig ein reelles Zwischenbild des Faserendes in der Nähe der Frontfläche des Hauptobjektivs. Üblicherweise liegt auch bei den derzeit eingesetzten Beleuchtungen für die Ophthalmologie dieses reelle Zwischenbild im Objektraum, und zwar ca. 50 mm unter dem Hauptobjektiv.

Die optischen Systemdaten für die Umfeld - Beleuchtung sind in Tabelle 2 aufgelistet:

### Systemdaten für Umfeld - Beleuchtungssystem

**Tabelle 2**

| **Nr.** | **Radius** (mm) | **Dicke** (mm) **Lichtleiter** | **Medium** | **freier Durchmesser** (mm) |
|---|---|---|---|---|
| 1 | -49.759 | 4.7 | Luft | 8.5 |
| 2 | -17.655 | 5.0 | NSK2 | 10.8 |
| 3 | -37.047 | 0.1 | Luft | 11.0 |
| 4 | 26.227 | 2.0 | NSF6 | 12.2 |
| 5 | -12.589 | 5.5 | NSK2 | 13.6 |
| 6 | Plan | 5.9 | Luft | **Blende** |
| 7 | -58.294 | 42.3 | Luft | 26.0 |
| 8 | -28.387 | 5.0 | NSK2 | 27.0 |
| 9 | 392.42 | 0.1 | Luft | 28.0 |
| 10 | 45.316 | 3.0 | NSF6 | 29.0 |
| 11 | -55.033 | 7.0 | NSK2 | 29.0 |
| 12 | Plan | 40.0 | Luft | Spiegel |
| 13 | 120.57 | 17.0 | Luft | 53.0 |
| 14 | -79.719 | 10.5 | NFK51 | 53.0 |
| 15 | -244.06 | 5.1 | NBAF4 | 53.0 |
| | | 193.6 | Luft | |
| | | **Sehfeld** | | |

Es kann durchaus auch sinnvoll sein, dem Anwender gleichzeitig eine Rotreflex- und eine Umfeldbeleuchtung zur Verfügung zu stellen. Dies kann beispielsweise dadurch erfolgen, dass die Plankonvexlinse 23 zwischen dem Kollektor 22 und den Blenden 24, 25 entweder auf einen transparenten Träger aufgekittet wird, oder beispielsweise als Kunststoffspritzgussteil (PMMA) mit einem entsprechenden transparentem Tragrand ausgebildet wird. Die Strahlen, welche die Plankonvexlinse 23 durchsetzen, erzeugen die Rotreflex Beleuchtung, die Strahlen die den Träger oder Tragrand durchsetzten, erzeugen die Umfeldbeleuchtung.

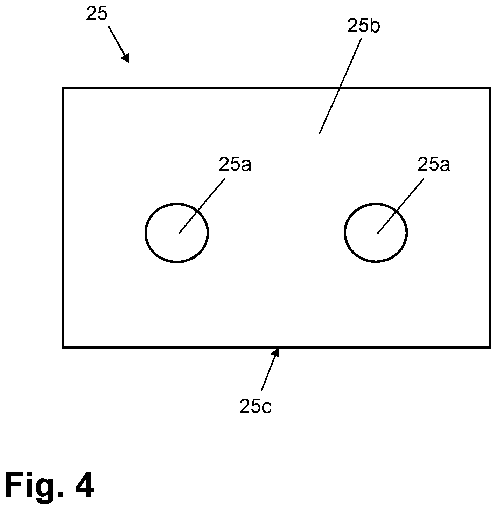

Um gleichzeitig die Rotreflex- und die Umfeldbeleuchtung zu erzeugen ist es ferner vorteilhaft, die Aperturblende 25 (Lochblende) in besonderer Weise zu gestalten. Ein Beispiel hierzu ist in Figur 4 dargestellt.

Gemäß Figur 4 kann die Aperturblende 25 vorteilhaft derart ausgestaltet sein, dass die Öffnungen 25a für die Rotreflexbeleuchtung beispielsweise eine hohe Transmission und die umliegenden Bereich 25b eine reduzierte (im Idealfall einstellbare) Transmission für die Umfeldbeleuchtung aufweisen.

Realisiert werden kann dies beispielsweise über ein transmissives oder reflektives LCD Display 25c, beziehungsweise über ein DMD Display.

### Bezugszeichenliste

- 10: Objektivelement
- 11: Mittel zum Aufteilen des Beleuchtungsstrahls
- 12: Beleuchtungsstrahlbündel
- 13: Beleuchtungsteilstrahlbündel
- 14: Vorrichtung zum Verändern des Strahlbündelquerschnitts

- 20: Beleuchtungseinrichtung
- 21: Lichtleiter
- 22: Kollektor
- 23: Plankonvexlinse
- 24: Leuchtfeldblende
- 25: Aperturblende
- 25a: Öffnung
- 25b: umliegender Bereich
- 25c: Display
- 26: optische Komponente
- 27: Umlenkelement
- 28: Objektivelement
- 29: Auge
- 30: Fundus
- 31: Sehfeld

## Patentansprüche

1. Operationsmikroskop für die Ophthalmo-Chirurgie, mit zwei stereoskopischen Beobachtungsstrahlengängen mit jeweils einem Beobachtungsstrahlbündel und mit einer Beleuchtungseinrichtung, aufweisend zumindest eine Lichtquelle zum Erzeugen von zwei Beleuchtungsstrahlbündeln (12) zum Beleuchten eines zu beobachtenden Auges, wobei die optischen Achsen der Beleuchtungsstrahlbündel (12) koaxial, das heißt unter exakt Null Grad oder in Form einer achsnahen Schrägbeleuchtung unter einem Winkel von kleiner/gleich 2 Grad zu den optischen Achsen der Beobachtungsstrahlbündel verlaufen, wobei die Beleuchtungsstrahlbündel (12) zwei Beleuchtungsspots auf dem Fundus des zu beobachtenden Auges bilden, die mittels zwei Lochblenden in der Zwischenbildebene mit einer Aperturblende erzeugt werden, wobei die Beleuchtungsspots eine runde Geometrie mit einem Durchmesser aufweisen, und wobei die Größe der Beleuchtungsspots auf dem Fundus das 1-fache, bevorzugt das 0.7-fache, weiter bevorzugt das 0.5-fache, besonders bevorzugt das 0.3-fache der Querschnittsfläche der Beobachtungsstrahlbündel auf dem Fundus nicht überschreitet.

2. Operationsmikroskop nach Anspruch , **dadurch gekennzeichnet, dass** die Durchmesser der Beleuchtungsspots so ausgebildet sind, dass sie auf dem Fundus des zu beobachtenden Objekts 1.5 mm, bevorzugst 1.0 mm, besonders bevorzugt 0.5 mm nicht überschreiten.

3. Operationsmikroskop nach einem der Ansprüche 1 oder 2, **dadurch gekennzeichnet, dass** die Beleuchtungseinrichtung ein Objektivelement (10) aufweist.

4. Operationsmikroskop nach einem der Ansprüche 1 bis 3, **dadurch gekennzeichnet, dass** Mittel vorgesehen sind, um jeweils ein Beobachtungsstrahlbündel und ein Beleuchtungsteilstrahlbündel (13) beziehungsweise das Beleuchtungsstrahlbündel (12) zu überlagern.

5. Operationsmikroskop nach Anspruch 1, **dadurch gekennzeichnet, dass** wenigstens eine Vorrichtung (14) zum Verändern des Bündelquerschnitts der Beleuchtungsstrahlbündel (12) und/oder der Beleuchtungsteilstrahlbündel (13) vorgesehen ist.

6. Operationsmikroskop nach Anspruch 5, **dadurch gekennzeichnet, dass** die Vorrichtung (14) als Blende, insbesondere Irisblende oder diskrete Blende, als LCD Display, als DMD, als LCOS oder als FLCOS ausgebildet ist.

7. Operationsmikroskop nach einem der Ansprüche 1 bis 7, **dadurch gekennzeichnet, dass** zwei oder mehr Lichtquellen vorgesehen sind und dass mittels jeder Lichtquelle ein Beleuchtungsteilstrahlbündel (13) erzeugt wird, oder dass eine einzige Lichtquelle vorgesehen ist und dass Mittel (11) zum Aufteilen des Beleuchtungsstrahlbündels (12) der Lichtquelle in die zwei oder mehr Beleuchtungsteilstrahlbündel (13) vorgesehen sind.

8. Operationsmikroskop nach einem der Ansprüche 1 bis 7, **dadurch gekennzeichnet, dass** die wenigstens eine Lichtquelle als Lampe, insbesondere als Halogenlampe oder Xenonlampe, als Laser, als nicht thermischer Strahler, als Lichtleiter, insbesondere als Faserlichtleiterbündel, als wenigstens eine LED oder wenigstens eine OLED ausgebildet ist.

9. Operationsmikroskop nach einem der Ansprüche 1 bis 8, **dadurch gekennzeichnet, dass** das Operationsmikroskop ein Hauptobjektivelement aufweist, welches identisch mit einem Objektivelement (10) der Beleuchtungseinrichtung ist, dass Mittel vorgesehen sind, um jeweils ein Beobachtungsstrahlbündel und ein Beleuchtungsteilstrahlbündel (13) beziehungsweise Beleuchtungsstrahlbündel (12) zu überlagern und dass die Mittel zum Überlagern derart angeordnet sind, dass eine Überlagerung von Beobachtungsstrahlbündel und Beleuchtungsteilstrahlbündel (13) beziehungsweise Beleuchtungsstrahlbündel (12) oberhalb des Hauptobjektivelements erfolgt.

10. Operationsmikroskop nach einem der Ansprüche 1 bis 8, **dadurch gekennzeichnet, dass** das Operationsmikroskop ein Hauptobjektivelement aufweist, welches identisch mit einem Objektivelement (10) der Beleuchtungseinrichtung ist, dass Mittel vorgesehen sind, um jeweils ein Beobachtungsstrahlbündel und ein Beleuchtungsteilstrahlbündel (13) beziehungsweise Beleuchtungsstrahlbündel (12) zu überlagern und dass die Mittel zum Überlagern derart angeordnet sind, dass eine Überlagerung von Beobachtungsstrahlbündel und Beleuchtungsteilstrahlbündel (13) beziehungsweise Beleuchtungsstrahlbündel (12) unterhalb des Hauptobjektivelements erfolgt.

## Claims

1. Surgical microscope for ophthalmic surgery, comprising two stereoscopic observation beam paths, each with an observation beam, and comprising an illumination device, comprising at least one light source for producing two illumination beams (12) for illuminating an eye to be observed, wherein the optical axes of the illumination beams (12) extend in coaxial fashion, i.e., at exactly zero degrees, or in the form of a near-axis oblique illumination at an angle of less than or equal to 2 degrees in relation to the optical axes of the observation beams, wherein the illumination beams (12) from two illumination spots on the fundus of the eye to be observed, said illumination spots being produced by means of two pinhole apertures in the intermediate image plane using an aperture stop, wherein the illumination spots have a round geometry with a diameter and wherein the size of the illumination spots on the fundus preferably does not exceed 1-times, preferably 0.7-times, more preferably 0.5-times, particularly preferably 0.3-times the cross-sectional area of the observation beams on the fundus.

2. Surgical microscope according to Claim, **characterized in that** the diameters of the illumination spots are embodied in such a way that they do not exceed 1.5 mm, preferably 1.0 mm, particularly preferably 0.5 mm on the fundus of the object to be observed.

3. Surgical microscope according to either of Claims 1 and 2, **characterized in that** the illumination device comprises an objective element (10).

4. Surgical microscope according to any one of Claims 1 to 3, **characterized in that** means are provided in order to superpose an observation beam and an illumination partial beam (13) or the illumination beam (12) in each case.

5. Surgical microscope according to Claim 1, **characterized in that** at least one apparatus (14) is provided for changing the beams cross section of the illumination beam (12) and/or the illumination partial beams (13).

6. Surgical microscope according to Claim 5, **characterized in that** the apparatus (14) is embodied as a stop, in particular as an iris stop or discrete stop, as an LCD display, as a DMD, as an LCOS or as an FLCOS.

7. Surgical microscope according to any one of Claims 1 to 7, **characterized in that** two or more light sources are provided and **in that** an illumination partial beam (13) is produced by means of each light source, or **in that** a single light source is provided and **in that** means (11) are provided for splitting the illumination beam (12) of the light source into the two or more illumination partial beams (13).

8. Surgical microscope according to any one of Claims 1 to 7, **characterized in that** the at least one light source is embodied as a lamp, in particular a halogen lamp or a xenon lamp, as a laser, as a non-thermal emitter, as a light guide, in particular as an optical fibre bundle, as at least one LED or as at least one OLED.

9. Surgical microscope according to any one of Claims 1 to 8, **characterized in that** the surgical microscope comprises a main objective element, which is identical to an objective element (10) of the illumination device, **in that** means are provided to superpose an observation beam and an illumination partial beam (13) or illumination beam (12) in each case and **in that** the means for superposition are arranged in such a way that a superposition of observation beam and illumination partial beam (13) or illumination beam (12) is implemented above the main objective element.

10. Surgical microscope according to any one of Claims 1 to 8, **characterized in that** the surgical microscope comprises a main objective element, which is identical to an objective element (10) of the illumination device, **in that** means are provided to superpose an observation beam and an illumination partial beam (13) or illumination beam (12) in each case and **in that** the means for superposition are arranged in such a way that a superposition of observation beam and illumination partial beam (13) or illumination beam (12) is implemented below the main objective element.

## Revendications

1. Microscope chirurgical pour la chirurgie ophtalmique, comportant deux trajets de faisceaux d'observation stéréoscopiques dont chacun comporte un faisceau lumineux d'observation et un dispositif d'éclairage, comportant au moins une source lumineuse destinée à générer deux faisceaux lumineux d'éclairage (12) pour l'éclairage d'un oeil à observer, dans lequel les axes optiques des faisceaux lumineux d'éclairage (12) sont coaxiaux, c'est-à-dire inférieurs à exactement zéro degré ou sous la forme d'un éclairage oblique proche de l'axe, sous un angle de 2 degrés ou moins par rapport aux axes optiques des faisceaux lumineux d'observation, dans lequel les faisceaux lumineux d'éclairage (12) forment deux points d'éclairage sur le fond d'oeil de l'oeil à observer, qui sont générés au moyen de deux diaphragmes à trous d'épingle dans le plan image intermédiaire avec un diaphragme d'ouverture, dans lequel les points d'éclairage présentent une géométrie circulaire ayant un certain diamètre, et dans lequel la taille des points d'éclairage sur le fond d'oeil ne dépasse pas 1 fois, de préférence 0,7 fois, et plus préférentiellement 0,5 fois, en particulier préférentiellement 0,3 fois la surface de la section transversale du faisceau lumineux d'observation sur le fond d'oeil.

2. Microscope chirurgical selon la revendication, **caractérisé en ce que** les diamètres des points d'éclairage sont conçus de manière à ce qu'ils ne dépassent pas 1,5 mm, de préférence 1,0 mm, en particulier préférentiellement 0,5 mm, sur le fond d'oeil de l'objet à observer.

3. Microscope chirurgical selon l'une des revendications 1 ou 2, **caractérisé en ce que** le dispositif d'éclairage comporte un élément d'objectif (10) .

4. Microscope chirurgical selon l'une des revendications 1 à 3, **caractérisé en ce qu'**il est prévu des moyens pour superposer respectivement un faisceau lumineux d'observation et un faisceau lumineux d'éclairage partiel (13) ou le faisceau lumineux d'éclairage (12).

5. Microscope chirurgical selon la revendication 1, **caractérisé en ce qu'**il est prévu au moins un dispositif (14) destiné à modifier la section transversale du faisceau lumineux d'éclairage (12) et/ou du faisceau lumineux d'éclairage partiel (13).

6. Microscope chirurgical selon la revendication 5, **caractérisé en ce que** le dispositif (14) est réalisé sous la forme d'un diaphragme, en particulier d'un diaphragme en iris ou d'un diaphragme discret, sous la forme d'un afficheur LCD, sous la forme d'un DMD, sous la forme d'un LCOS ou sous la forme d'un FLCOS.

7. Microscope chirurgical selon l'une des revendications 1 à 7, **caractérisé en ce qu'**il est prévu deux sources lumineuses ou plus et **en ce qu'**un faisceau lumineux d'éclairage partiel (13) est généré au moyen de chaque source lumineuse, ou **en ce qu'**il est prévu une source lumineuse unique et **en ce qu'**il est prévu des moyens (11) destinés à diviser le faisceau lumineux d'éclairage partiel (12) provenant de la source lumineuse en deux faisceaux lumineux d'éclairage partiels (13) ou plus.

8. Microscope chirurgical selon l'une des revendications 1 à 7, **caractérisé en ce que** ladite au moins une source lumineuse est réalisée sous la forme d'une lampe, en particulier sous la forme d'une lampe à halogène ou d'une lampe au xénon, sous la forme d'un laser, sous la forme d'un élément rayonnant non thermique, sous la forme d'un guide de lumière, en particulier sous la forme d'un faisceau de fibres optiques, sous la forme d'au moins une LED ou d'au moins une OLED.

9. Microscope chirurgical selon l'une des revendications 1 à 8, **caractérisé en ce que** le microscope chirurgical comporte un élément d'objectif principal qui est identique à un élément d'objectif (10) du dispositif d'éclairage, **en ce qu'**il est prévu des moyens pour superposer respectivement un faisceau lumineux d'observation et un faisceau lumineux d'éclairage partiel (13) ou un faisceau lumineux d'éclairage (12) et **en ce que** les moyens de superposition sont agencés de manière à ce qu'une superposition du faisceau lumineux d'observation et du faisceau lumineux d'éclairage partiel (13) ou du faisceau lumineux d'éclairage (12) ait lieu au-dessus de l'élément d'objectif principal.

10. Microscope chirurgical selon l'une des revendications 1 à 8, **caractérisé en ce que** le microscope chirurgical comprend un élément d'objectif principal qui est identique à un élément d'objectif (10) du dispositif d'éclairage, **en ce qu'**il est prévu des moyens pour superposer respectivement un faisceau lumineux d'observation et un faisceau lumineux d'éclairage partiel (13) ou un faisceau lumineux d'éclairage (12) et **en ce que** les moyens de superposition sont agencés de manière à ce qu'une superposition du faisceau lumineux d'observation et du faisceau lumineux d'éclairage partiel (13) ou du faisceau lumineux d'éclairage (12) ait lieu en-dessous de l'élément d'objectif principal.
